# EUROPEAN PATENT APPLICATION

(11) **EP 2 620 504 A1**
(43) Date of publication of application: **31.07.2013**
(21) Application number: 12152454.0
(22) Date of filing: 25.01.2012
(51) Int. Cl.: C12P 7/04, C12P 7/24, C12P 7/40

(54) **Process for oxidizing alkenes employing the Pseudomonas putida GPo1 AlkB monooxygenase**

(71) Applicant: Evonik Industries AG, 45128 Essen (DE)
(72) Inventor: Engel, Philip, 45147 Essen (DE); Haas, Dr Thomas, 48161 Münster (DE); Pfeffer, Jahn Christoph, 45355 Essen (DE); Thum, Oliver, 40880 Ratingen (DE); Gehring, Christian, 45770 Marl (DE)

(57) **Abstract**

The present invention relates to method for oxidising an alkene comprising contacting said alkene with an AlkB-type oxidoreductase in the presence of oxygen, and a use of an AlkB-type oxidoreductase, preferably AlkB from *Pseudomonas putida* GPO1 or a variant thereof, for oxidising an alkene to an alcohol and/or acid.

## Description

The present invention relates to a method for oxidising an alkene comprising contacting said alkene with a monooxygenase, preferably an AlkB-type oxidoreductase in the presence of oxygen, and a use of a monooxygenase, preferably an AlkB-type oxidoreductase, more preferably AlkB from *Pseudomonas putida* GPO1 or a variant thereof, for oxidising an alkene to an alcohol and/or acid.

Alkenes, also referred to in organic chemistry as olefins, are hydrocarbons that comprise at least one carbon-carbon double bond. In line with their unsaturated character, alkenes are more reactive than alkanes, albeit similar in terms of many physical properties.

Alkenes are often obtained by cracking fossil fuels such as natural gas condensate components comprising alkanes at high temperatures and separating from the mixture of products aliphatic alkenes of interest. Other methods include elimination reactions such as the dehydration of alcohols and hydrogenation of alkynes.

Alkenes are often used as building blocks for synthetic polymers. Such polymers, for example polyethylene and polypropylene, are of high industrial value. Polymerization of alkenes may be initiated by addition of a catalyst, for example benzoyl peroxide, which comprises or is converted to a reactive radical and attacks a monomer, generating another radical which may attack, in a second step, another monomer for elongation of the chain. This goes on and on until propagation of the chain is terminated, for example by reaction with another elongating chain, by interaction with a radical inhibitor such as oxygen or radical disproportionation, the latter yielding a saturated and an unsaturated polymer product. Shortcomings of such approaches involve, in addition to dependency on fossil fuels, the need to use hazardous and instable chemicals.

While any alkene, even ethylene, the simplest possible alkene, may be subjected to such a polymerisation, it is often desirable to produce more sophisticated polymers comprising more complex alkene building blocks. Hence, there is the need to convert alkenes to substituted alkenes, for example by oxidation to introduce hydroxyl or carboxy groups, prior to subjecting them to polymerisation.

The state of the art teaches various reactions that may be used to oxidise alkenes, for example ozonolysis, the cleavage of an alkene to form organic compounds such as carboxylic acids and aldehydes. However, if an alkene is to be oxidised for the purpose of producing polymer building blocks, care must be taken that the double bond is left intact or else the product can no longer be used for polymerisation reactions based on radical mechanisms. In other words, the oxidation reaction must be highly selective.

Therefore, the problem underlying the present invention is to provide a method for converting alkenes to oxidised alkenes such as unsaturated alcohols, aldehydes and carboxylic acids, wherein the alkene need not be subjected to harsh reaction conditions such as highly oxidative species, for example ozone and hydrogen peroxide, strong acids such as tosilic acid, or to metal catalysts, for example catalysts based on osmium.

Another problem underlying the present invention is to provide a method for the production of oxidised alkenes, in particular methacrylic acid, under gentle conditions, wherein the oxidation is selective in the sense that a specific proportion of alcohol, aldehyde and carboxylic acid is produced, while the double bond is essentially left intact and/or a smaller proportion of side-products is generated.

In a first aspect, the problem underlying the present invention is solved by a method for oxidising an alkene comprising contacting said alkene with a monooxgenase in the presence of oxygen.

In a first embodiment, the problem is solved by a method, wherein the monooxygenase is an AlkB-type oxidoreductase.

In a second embodiment, which is also an embodiment of the first embodiment, the problem is solved by a method, wherein the AlkB-type oxidoreductase is AlkB from *Pseudomonas putida* GPO1 or a variant thereof.

In a third embodiment, which is also an embodiment of the first to second embodiments, the problem is solved by a method, wherein the alkene is represented by formula (I)

R¹R²C = C R³R⁴ (I),

wherein R¹, R², R³ and R⁴ is each and independently selected from the group comprising H-(CH₂)ₓ-, wherein x is 0 or more, wherein two out of R¹, R² and R³ may form cyclic alkyls, preferably alkyls comprising five or six members, and wherein preferably R¹, R² and R³ is each and independently selected from the group comprising H-(CH₂)ₓ- and x is 1 to 24.

In a fourth embodiment, which is also an embodiment of the first to third embodiments, R⁴ is methyl.

In a fifth embodiment, which is also an embodiment of the first to fourth embodiments, the problem is solved by a method, wherein R¹ = R² = hydrogen or R¹ = R³ = hydrogen, and preferably R¹ = R² = R³ = hydrogen.

In a 6^{th} embodiment, which is also an embodiment of the first to 5^{th} embodiments, the problem is solved by a method, wherein R¹ = R² = hydrogen and R³ is methyl.

In a 7^{th} embodiment, which is also an embodiment of the first to 6^{th} embodiments, the problem is solved by a method, wherein R¹ = R² = hydrogen and R³ is -CH=CH₂.

In an 8^{th} embodiment, which is also an embodiment of the first to 7^{th} embodiments, the problem is solved by a method, wherein the alkene is gaseous at room temperature and under atmospheric pressure.

The problem underlying the present invention is solved, in a second aspect, by a use of a monooxygenase, preferably an AlkB-type oxidoreductase, more preferably AlkB from *Pseudomonas putida* GPO1 or a variant thereof, for oxidising an alkene to an alcohol and/or acid.

In a first embodiment of the second aspect, the problem is solved by a use, wherein the alkene is represented by formula (I)

R¹R²C = C R³R⁴ (I),

wherein R¹, R² and R³ is each and independently selected from the group comprising H-(CH₂)ₓ-, wherein x is 0 or more, wherein two out of R¹, R² and R³ may form cyclic alkyls, preferably alkyls comprising five or six members, and wherein preferably R¹, R² and R³ is each and independently selected from the group comprising H-(CH₂)ₓ- and x is 1 to 24.

In a second embodiment of the second aspect, which is also an embodiment of the first embodiment, the problem is solved by a use, wherein R⁴ is methyl.

In a third embodiment of the second aspect, which is also an embodiment of the first to second embodiments, the problem is solved by a use, wherein R¹ = R² = hydrogen or R¹ = R³ = hydrogen, and preferably R¹ = R² = R³ = hydrogen.

In a 4^{th} embodiment of the second aspect, which is also an embodiment of the first to third embodiments, the problem is solved by a use, wherein R¹ = R² = hydrogen and R³ is methyl.

In a 5^{th} embodiment of the second aspect, which is also an embodiment of the first to 4^{th} embodiments, the problem is solved by a use, wherein R¹ = R² = hydrogen and R³ is -CH=CH₂.

In a 6^{th} embodiment of the second aspect, which is also an embodiment of the first to 5^{th} embodiments, the problem is solved by a use, wherein the alkene is gaseous at room temperature and under atmospheric pressure.

In a 7^{th} embodiment of the second aspect, which is also an embodiment of the first to 8^{th} embodiments and of the first aspect and all of its embodiments, the problem is solved by a use or method, wherein the alkene is oxidised to a mixture of oxidation products, and the proportion of acid produced is more than 25, preferably more than 40 % of the entirety of oxidation products produced.

In an 8^{th} embodiment of the second aspect, which is also an embodiment of the first to 7^{th} embodiments and of the first aspect and all of its embodiments, the problem is solved by a use or method, wherein the oxidoreductase is provided as a whole cell catalyst expressing said oxidoreductase.

The present invention is based on the surprising finding that AlkB-type oxidoreductases, a group of enzymes previously known only as capable of oxidising alkanes and saturated alkyl groups, may be used to oxidise alkenes, in particular gaseous alkenes.

The inventive method and use centers around contacting alkB-type oxidoreductases with alkenes. The archetype of this class of oxidoreductases, AlkB, is an redox protein from the *Pseudomonas putida* AlkBGT system, dependent on two auxiliary polypeptides, AlkG and AlkT. AlkT is a FAD-dependent rubredoxin reductase transferring electrons from NADH to AlkG. AlkG is a rubredoxin, an iron-containing redox protein functioning as a direct electron donor to AlkB. In a preferred embodiment, the term "alkB-type oxidoreductases", as used herein, refers to a rubredoxin-dependent oxidoreductase having a hydrocarbon binding site. In another preferred embodiment, the term "hydrocarbon binding site", as used herein, refers to a stretch of the protein surface capable of binding to a hydrocarbon, preferably an alkane and/or alkene, more preferably within reach of the catalytic centre of the protein. In another preferred embodiment, the term "rubredoxin-dependent alkane oxidase", as used herein refers to an oxidoreductase that recognises as its substrate an alkane receiving electrons via a rubredoxin, the latter being, in a more preferred embodiment, an iron-sulphur protein having an α+β class fold with 2 α helices and 2 to 3 β-strands transferring electrons to the alkane oxidase. In a most preferred embodiment, the alkB-type oxidoreductase is AlkB from *Pseudomonas putida* Gpo1 (Access code: CAB54050.1, any access code used in the application refers to the respective sequence from the Genbank database run by the NCBI, wherein the release referred to is the one available online on the 15^{th} January, 2012) or a variant thereof.

In a preferred embodiment, the term "contacting", as used herein, means bringing about direct contact between alkene and oxidoreductase such that the latter is able to oxidise the former. For example, the cell and the alkene may not be in different compartments separated by a membrane such as an inorganic membrane impermeable for the cell and the alkene of interest. If the alkene is solid or soluble, it may simply be added to the oxidoreductase in an aqueous solution. If the alkene is gaseous, the aqueous solution comprising the cell may be sparged with a gas comprising said gaseous alkene.

In case the alkene is solid, it may be solubilised using suitable organic solvents and then be added to the aqueous solution. Preferred organic solvents are biocompatible solvents, i.e. solvents that allow for the viability of the cell used and/or activity of the alkB-type oxidoreductase chosen. In a preferred embodiment, the organic solvent is a fatty acid comprising 5 or more, more preferably 8 or more, most preferably 12 or more carbon atoms or derivatives thereof such as alkyl esters. In a preferred embodiment, the organic solvent is lauric acid methyl ester.

The alkB-type oxidoreductase may be a recombinant oxidoreductase. In a preferred embodiment, the term "recombinant" alkB-type oxidoreductase, as used herein, means that the nucleic acid encoding the alkB-type oxidoreductase is not an endogenous nucleic acid of the organism used to express it, or indeed any wild type organism, but has been made using genetic engineering. The person skilled in the art is familiar with suitable plasmids, nucleic acid elements, for example promoter sequences and methods that may be used to make such plasmids. Standard molecular biology methods are described, for example, in Sambrook et al. (Molecular Cloning - A Laboratory Manual (1989) Cold Spring Harbor Laboratory Press).

Although a wild type strain expressing an alkB-type oxidoreductase may in principle be used, it is preferred that the alkB-type oxidoreductase is overexpressed. In a preferred embodiment, the term "overexpressed", as used herein, means that the concentration of the polypeptide in questions, compared to its concentration in the respective wild type cell, is elevated. The person skilled in the art is familiar with techniques that may be used for overexpressing a alkB-type oxidoreductase, for example the use of the pET or pGEX system of plasmids.

The alkB-type oxidoreductase may be a purified or isolated polypeptide. In a preferred embodiment, the term "purified", as used herein means that the polypeptide referred to as such is purer than at the time of its expression in a cell. The purity may be judged by Polyacrylamide gel electrophoresis followed by Coomassie blue staining of the gel produced. In a preferred embodiment, the polypeptide is more than 50, 60, 70, 80, 90, 95 or 99 % pure, but it may in principle be used in any degree of purity, from crude cell lysate to 100 % pure polypeptide. The person skilled in the art is familiar with protein purification methods, for example affinity chromatography, ammonium sulphate precipitation and gel filtration chromatography.

The alkene is contacted with the alkB-type oxidoreductase in an environment compatible with alkB-type oxidoreductase activity. The person skilled in the art is familiar with standard parameters, such as ionic strength, temperature and the composition of suitable aqueous buffers that need to be considered with respect to enzyme activity and is capable of determining suitable conditions within the scope of routine experimentation. Suitable conditions for maintaining alkB-type oxidoreductase activity are described, for example, in Grant C., Woodley, J. M, and Baganz, F (2011) Enzyme and Microbial Technology 48, 480-486.

It is essential that oxygen is present as long as the oxidation of the alkene catalysed by the alkB-type oxidoreductase continuous. Typically, the oxidoreductase is in an aqueous solution and oxygen is introduced by stirring the reaction vessel under aerobic conditions, i.e. in the presence of molecular oxygen (O₂). Alternatively, the solution may be sparged with pure molecular oxygen or gas mixtures comprising molecular oxygen, preferably in addition to inert gases such as nitrogen or argon. If the alkene to be oxidised is gaseous under the conditions contemplated, it may be part of such a mixture. In a preferred embodiment, the aqueous solution is in contact with air to provide oxygen.

In a preferred embodiment, molecular oxygen may be present at a partial pressure exceeding atmospheric pressure, preferably measured at room temperature (25 °C), preferably at more than 1,5, 2, 3, 4, 5, 6, 7, 8, 9, or 10 bar.

The teachings of the present invention may not only be carried out using biological macromolecules having the exact amino acid or nucleic acid sequences referred to in this application explicitly, for example by name or accession number, or implicitly, but also using variants of such sequences. In a preferred embodiment, the term "variant", as used herein, comprises amino acid or nucleic acid sequences, respectively, that are 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease is capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Stringency of hybridisation reactions is readily determinable by one of ordinary skilled in the art, and in generally is an empirical calculation dependent on probe length, washing temperature and salt concentration.

In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc.,. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

Alternatively, the alkB-type oxidoreductase may be provided as a whole cell biocatalyst. In a preferred embodiment, the term "whole cell biocatalyst", as used herein, refers to a viable cell that expresses the alkB-type oxidoreductase in a manner that allows contacting the latter with the alkene. In a preferred embodiment, the alkB-type oxidoreductase is located at the outer cell membrane and so exposed to the aqueous solution surrounding the whole cell biocatalyst. Alternatively, the alkB-type oxidoreductase is expressed inside the cell.

In a preferred embodiment, the whole cell biocatalyst expresses, in addition to the alkB-type oxidoreductase, the polypeptide encoded by alkL from *Pseudomonas putida* (Access code CAB69081.1) or a variant thereof.

The inventive teachings may be carried out using a wide range of cells. In a preferred embodiment, the term "cell", as used herein, refers to any permanently unicellular cell comprising bacteria, archaea, fungi, algae and the like. In a preferred embodiment, the cell is a bacterial cell, more preferably one from the group comprising *Pseudomonas, Corynebacterium* and *Escherichia,* most preferably *Escherichia coli.* In another preferred embodiment, the cell is a lower eukaryote, more preferably a fungus from the group comprising *Saccharomyces, Candida, Picchia, Schizosaccharomyces* and *Yarrowia,* and is most preferably *Saccharomyces cerivisiae.* Throughout this application, the term "cell" is used synonymously and interchangeably with the term "microorganism". The cell may be an isolated cell, in other words a pure culture of a single strain of cell, or may comprise a mixture of at least two strains. Biotechnologically relevant cells are commercially available, for example from the American Type Culture Collection (ATCC) or the German Collection of Microorganisms and Cell Cultures (DSMZ). Protocols for keeping and modifying microorganisms are available from the prior art, for example Sambroke/Fridge/Maniadis (1989): Molecular cloning - A Laboratory Manual, Cold Spring Harbour Press, 2nd editi*on,* Fuchs/Schlegel (2007), Allgemeine Mikrobiologie, 2008, Georg Thieme Verlag*.*

The inventive method comprises contacting the alkene with an aqueous solution comprising alkB-type oxidoreductase. This step may not only comprise temporarily contacting the alkene with the solution, but in fact incubating the alkene in the presence of the alkB-type oxidoreductase sufficiently long to allow for an oxidation reaction to occur, for example for at least 1, 2, 4, 5, 10 or 20 hours. The temperature chosen must be such that the inventive cells remains catalytically competent and/or metabolically active, for example 10 to 42 °C, preferably 30 to 40 °C, most preferably 32 to 38 °C in case the inventive cell is an *E. coli* cell.

The alkene to be oxidised may be any alkene. In a preferred embodiment, the term "alkene", as used herein, refers to any compound represented by formula Rₓ-CH=CH-CR_{y}H₂, wherein Rₓ is any chemical group and R_{y} is hydrogen or forms, together with or Rₓ or parts of it, an alkylene ring. In another preferred embodiment, the term "alkene", as used herein, refers to a hydrocarbon represented by the formula CₙH₂ₙ, wherein n is or is more than 3, 4, 5, 6, 7, 8, 9, 10, 11, 12, 13, 14, 15, 16, 17, 18, 19, 20, 21, 22, 23, 24, 25, 26, 27, 28, 29, 30. In another preferred embodiment, the alkene is a cycloalkene represented by the formula CₙH₂ₙ₋₂, wherein n is or is more than 5, 6, 7, 8, 9, 10, 11, 12, 13, 14, 15, 16, 17, 18, 19, 20, 21, 22, 23, 24, 25, 26, 27, 28, 29, 30. In a preferred embodiment, the alkene is, at room temperature (25 °C) and atmospheric pressure, a gaseous alkene, for example isobutene.

A gaseous alkene is preferably present at a partial pressure, preferably measured at room temperature (25 °C), exceeding atmospheric pressure, for example at more than 1,5, 2, 3, 4, 5, 6, 7, 8, 9 or 10 bar. The total combined pressure of all gaseous compounds present, for example oxygen, gaseous alkenes and nitrogen, may be more than 1,5, 2, 3, 4, 5, 6, 7, 8, 9 or 10 bar.

The term "an aqueous solution" comprises any solution comprising, as main solvent, water, that may be used to keep the whole cell biocatalyst expression the alkB-type oxidoreductase, at least temporarily, in a metabolically active and/or viable state or the alkB-type oxidoreductase catalytically competent and comprises, if such is necessary, any additional substrates. The person skilled in the art is familiar with numerous aqueous solutions, usually referred to as media, that may be used to grow and sustain cells, for example LB medium in the case of *E*. *coli.* In a preferred embodiment the aqueous solution is kept under aerobic conditions. It is advantageous to use for growing cells to be used as whole cell biocatalysts a full medium owing to the increase in growth rate compared to minimal medium.

It is advantageous to use as an aqueous solution for carrying out the inventive method or use a simple buffer or minimal medium, *i.e*. a medium of reasonable simple composition that comprises only the minimal set of salts and nutrients indispensible for keeping the cell in a metabolically active and/or viable state, by contrast to complex mediums. For example, M9 medium may be used as a minimal medium. If the alkyl to be oxidised has limited solubility in water, a detergent such as Tween or Triton may be added to the aqueous solution or a hydrophobic solvent may be used to solubilise the alkyl to be oxidised. The person skilled in the art is familiar with the preparation of various aqueous and organic solutions.

The reaction may be run in a batch mode or in a continuous mode. The person skilled in the art is familiar with adequate fermenters and/or reaction vessels.

Following the inventive teachings it is possible to oxidise alkenes such that a specified proportion of oxidation products is produced. In a preferred embodiment, the term "proportion of acid produced is more than X % of the entirety of oxidation products produced", as used herein, means that X % of the alkene molecules oxidised by the AlkB-type oxidoreductase are converted to the respective acid rather than to the respective alcohol or aldehyde. For example, the proportion of acid produced is more than 50 % of the entirety of oxidation products if one mol of isobutene is contacted with an AlkB-type oxidoreductase, 0,5 mol is oxidised by the oxidoreductase, and 0,25 is oxidised to yield methacrylic acid. In a preferred embodiment, the proportion of acid produced is more than 10, 20, 30, 40, 50, 60, 70, 80 or 90 % of the entirety of oxidation products produced. In a preferred embodiment, the proportion of acid produced is determined after the reaction has essentially reached equilibrium if the reaction is run in a batch reaction mode or is determined considering the molecules leaving the reaction vessel run if the reaction is run in a continuous mode.

The invention is further illustrated by the following figures and non-limiting examples from which further features, embodiments, aspects and advantages of the present invention may be taken.
**Fig. 1** shows the concentration of 2-methyl-2-propene-1-ol over time as observed in Example 1.
**Fig. 2** shows the concentration of methacrylic acid over time as observed in Example 1.

### Example 1: Oxidation of Isobutene using E. coli W3110 comprising monooxygenase (AlkBGT) from Pseudomonas putida GPO1.

### a) Production of biomass at a 10 I scale

Preseed culture: 1 L of LB medium (5 g/l yeast extract, 10 g/l peptone, 0,5 g/l NaCl, solved in 1 L water, autoclaved for 20 minutes at 121 °C) was prepared.

25 ml each of this solution were transferred into five 100 ml flasks with baffles, complemented with 25 µl of a kanamycin solution (50 g/l) sterilized by filtration and inoculated using 200 ml each of a glycerol cryoculture of *E*. *coli* W3110 pBT 10 (WO2009/677461). These cultures were incubated at 37 °C and 200 rpm (amplitude 2.5 cm) for 18 hours.

Seed culture: 1 L of high density cell medium (HCD-medium) was prepared, comprising 1.76 g/l NH₄SO₄, 19.08 g/l K₂HPO₄, 12.5 g/l KH₂PO₄, 6.66 g/l yeast extract, 1.96 g/l sodium-citrate, 17 ml NH₄Fe citrate solution (1 %), 5 ml trace element solution US3 (1 L of the trace element solution US 3 comprises 36.5 g HCl 37 %, 1.91 g MnCl₂ x 4H₂O, 1.87 g ZnSO₄ x 7H₂O, 0.8 g sodium EDTA x 2H₂O, 0.3 g H₃BO₃, 0.25 g Na₂MoO₄ x 2H₂O, 4.7 g CaCl₂ x 2H₂O, 17.8 g FeSO₄ x 7 H₂O, 0.15 g CuCl₂, solved in 1 L water.), 30 ml of feed solution (glucose 50 % w/v, MgSO₄ x 7 H₂O 0.5 % w/v, NH₄Cl 2.2 % w/v), 1 ml of kanamycin solution (50 g/l). 948 ml solution comprising NH₄SO₄ to Na₃-citrate were autoclaved, the other components were sterilized by filtration separately and added subsequently. The pH was 6.8.

5 x 75 ml of the HCD medium was transferred into 1000 ml shake flask with baffles, inoculated with 25 ml preseed culture each and incubated for 30 h at 37 °C and 200 rpm (amplitude 2.5 cm).

A sterile 10 L fermenter was set up using 7 L of sterile medium comprising 1.75 g/l (NH₄)₂SO₄, 19 g/l K₂HPO₄ x 3 H₂O, 12.5 g/l KH₂PO₄, 6.6 g/l yeast extract, 2.24 g/l Na₃-citrate x 2H₂O, 15 g/l glucose, 0.49 g/l MgSO₄ x 7 H₂O, 16.6 ml/LNH₄Fe citrate solution (1 % w/v), 15 ml/I trace element solution (as in the seed culture) 1 ml/L kanamycin solution (50 mg/l) and 2 ml anti foam reagent (Delamex). As a feed, an autoclaved solution of glucose (50 % w/v) comprising MgSO₄ x 7H₂O 10g/l was connected, and 0.5 M H₂SO₄ and 25 % NH₄OH was used for pH correction.

The cultures from the shake flasks were pooled in a sterile manner and used to inoculate the fermenter *via* a transfer flask. The fermentation conditions were: pO₂ 30 %, air flow 6 nlpm, stirrer 400 - 1200 rpm temperature 37 °C, pH 7, feed start 8 h, feed rate 150 - 250 g/h. After 19 h the temperature was lowered to 30 °C, and the culture was induced using 0.4 mM DCPK. After 23 h the OD₆₀₀ in the fermenter was approximately 100, the culture broth was removed in a sterile manner and spun down at 8000 rpm in 1000 ml centrifuge flasks comprising 500 ml solution each. The supernatant was discarded, and 10 g pellets were aliquoted in falcon tubes. The pellets could immediately be for biotransformation or could be frozen for further use a - 80 °C.

### b) Biotransformation of Isobutene

Three of the biomass pellets prepared as described in section a) were resuspended in 50 ml 70 mM ammonium phosphate buffer at pH 7 each (composition: 8 g/L (NH₄)H₂PO₄, 0.5 g/L NaCl, 0.49g/L MgSO₄ x 7H₂O, 1 ml trace element solution US3 and 50 µg/l kanamycin). 5 % NH₄OH was used to adjust the pH.

150 ml ammonium phosphate buffer comprising approximately 3 drops of autoclaved anti foam reagent (Delamex) was transferred into each of three sterile 300 ml fermenters. A gas mixture comprising 25 % Isobutene and 75 % synthetic air from a gas cylinder at an initial pressure of 6 bar was introduced into the fermenters via a metal interperlator having a pore size of 0.2 µm at a flow rate of approximately 15 Nl/h. The temperature of the fermenters was maintained at 40 °C using a water bath, and their contents were stirred using a magnetic stirrer at 900 rpm. The outgoing air was passed through washing bottles comprising 150 ml water each.

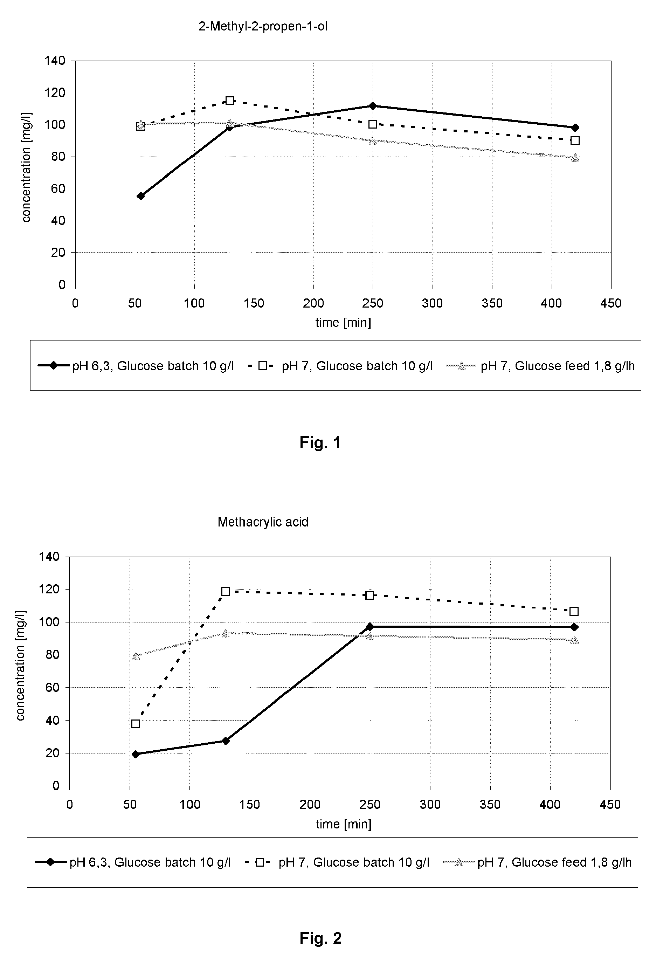

Fermenters were inoculated using 50 ml of the resuspended biomass pellets each *via* the sample-taking tube. Glucose was either added as a batch (10 g/L) or fed continuously (1.8 g/lh). PH was maintained at 7.0 or 6.3, respectively, using 2.5 % ammonia water. A 6 ml sample was removed from the fermenter after 55, 130, 250 and 420 minutes each. Samples were spun for 10 minutes at 10000 g and room temperature and the supernatant was filtered. Chromatographic analysis was carried out using HPLC using refractive indexdetector and diode-array-detection HPLC as part of an Agilent techonologies 1200 facility. An Aminex HPX-87H-column (300mmx 7.8 mm) was used. The facility was run using 10 mM H₂SO₄ as eluent at a flow rate of 0.6 ml/min and a column temperature of 40 °C. Standards of all compounds to be analyzed were prepared in ultrapure water and measured under identical conditions. Evaluation was carried out by comparing retention times. The concentration of 2-methyl-2-propene-1-ol and methacrylic acid over time is depicted in **Figs. 1** and **2**.

## Claims

1. A method for oxidising an alkene comprising contacting said alkene with a monooxygenase in the presence of oxygen.

2. The method according to claim 1, wherein the monooxygenase is an AlkB-type oxidoreductase.

3. The method according to claim 2, wherein the AlkB-type oxidoreductase is AlkB from *Pseudomonas putida* GPO1 or a variant thereof.

4. The method according to any of claims 1 to 3, wherein the alkene is represented by formula (I)
R¹R²C = C R³R⁴ (I),
wherein R¹, R², R³ and R⁴ is each and independently selected from the group comprising H-(CH₂)ₓ-, wherein x is 0 or more, wherein two out of R¹, R² and R³ may form cyclic alkyls, preferably alkyls comprising five or six members, and wherein preferably R¹, R² and R³ is each and independently selected from the group comprising H-(CH₂)ₓ- and x is 1 to 24

5. The method according to any of claims 1 to 3, wherein R⁴ is methyl.

6. The method according to claim 5, wherein R¹ = R² = hydrogen or R¹ = R³ = hydrogen, and preferably R¹ = R² = R³ = hydrogen.

7. The method according to claim 5, wherein R¹ = R² = hydrogen and R³ is methyl.

8. The method according to claim 7, wherein R¹ = R² = hydrogen and R³ is -CH=CH₂.

9. The method according to any of claims 1 to 8, wherein the alkene is gaseous at room temperature and under atmospheric pressure.

10. A use of a monooxygenase, preferably an AlkB-type oxidoreductase, more preferably AlkB from *Pseudomonas putida* GPO1 or a variant thereof, for oxidising an alkene to an alcohol and/or acid.

11. The use according to claim 10, wherein the alkene is represented by formula (I)
R¹R²C = C R³R⁴ (I),
wherein R¹, R² and R³ is each and independently selected from the group comprising H-(CH₂)ₓ-, wherein x is 0 or more, wherein two out of R¹, R² and R³ may form cyclic alkyls, preferably alkyls comprising five or six members, and wherein preferably R¹, R² and R³ is each and independently selected from the group comprising H-(CH₂)ₓ- and x is 1 to 24.

12. The use according to claim 9, wherein R⁴ is methyl.

13. The use according to claim 12, wherein R¹ = R² = hydrogen or R¹ = R³ = hydrogen, and preferably R¹ = R² = R³ = hydrogen.

14. The use according to claim 13, wherein R¹ = R² = hydrogen and R³ is methyl.

15. The use according to claim 14, wherein R¹ = R² = hydrogen and R³ is -CH=CH₂.

16. The method according to any of claims 1 to 9 or the use according to any of claims 10 to 15, wherein the alkene is gaseous at room temperature and under atmospheric pressure.

17. The method according to any of claims 1 to 9 or 16 or the use according to any of claims 10 to 16, wherein the alkene is oxidised to a mixture of oxidation products, and the proportion of acid produced is more than 25, preferably more than 40 % of the entirety of oxidation products produced.

18. The method according to any of claims 1 to 9 and 16 to 17 or the use according to any of claims 10 to 16, wherein the oxidoreductase is provided as a whole cell catalyst expressing said oxidoreductase.
